Europäisches Patentamt

European Patent Office

Office européen des brevets

(11) Veröffentlichungsnummer: **0 321 749**

**A1**

## (12) EUROPÄISCHE PATENTANMELDUNG

(21) Anmeldenummer: 88119979.8

(22) Anmeldetag: 30.11.88

(51) Int. Cl.⁴: **C07K 15/06 , C12N 5/00 , C12N 15/00 , C12P 21/00 , G01N 33/68 , //(C12P21/00, C12R1:91)**

Ein Antrag gemäss Regel 88 EPÜ auf Berichtigung der Beschreibung liegt vor. Über diesen Antrag wird im Laufe des Verfahrens vor der Prüfungsabteilung eine Entscheidung getroffen werden (Richtlinien für die Prüfung im EPA, A-V, 2.2).

(30) Priorität: 03.12.87 DE 3741007
10.08.88 DE 3827145

(43) Veröffentlichungstag der Anmeldung:
28.06.89 Patentblatt 89/26

(84) Benannte Vertragsstaaten:
**BE CH DE FR GB IT LI NL SE**

(71) Anmelder: **Gesellschaft für Biotechnologische Forschung mbH (GBF) Mascheroder Weg 1 D-3300 Braunschweig-Stöckheim(DE)**

(72) Erfinder: **Böldicke, Thomas, Dr. Dipl.-Chem.**
Mascheroder Weg 1
D-3300 Braunschweig(DE)
Erfinder: **Kindt, Silke**
Mascheroder Weg 1
D-3300 Braunschweig(DE)
Erfinder: **Maywald, Friedhelm, Dr. Dipl.-Bio.**
Mascheroder Weg 1
D-3300 Braunschweig(DE)
Erfinder: **Fitzlaff, Gaby**
Mascheroder Weg 1
D-3300 Braunschweig(DE)
Erfinder: **Frank, Ronald, Dr. Dipl.-Chem.**
Mascheroder Weg 1
D-3300 Braunschweig(DE)
Erfinder: **Collins, John, Prof. Dr.**
Mascheroder Weg 1
D-3300 Braunschweig(DE)

(74) Vertreter: **Boeters, Hans Dietrich, Dr. et al Boeters & Bauer, Patentanwälte Thomas-Wimmer-Ring 14 D-8000 München 22(DE)**

(54) **Monoklonale Antikörper, Hybridoma-Zellinien zur Gewinnung der Antikörper, Verfahren zur Gewinnung der Antikörper und der Zellinien, sowie Verwendung der Antikörper.**

(57) Die Erfindung betrifft monoklonale Antikörper gegen Protein-Partialsequenzen oder Proteine, Hybridoma-Zellinien zur Gewinnung der Antikörper, Verfahren zur Gewinnung der Antikörper und der Hybridoma-Zellinien sowie die Verwendung der monoklonalen Antikörper zur quantitativen Bestimmung der Proteine in Körperflüssigkeiten und zur Tumordiagnose.

EP 0 321 749 A1

## Monoklonale Antikörper, Hybridome-Zellinien zur Gewinnung der Antikörper, Verfahren zur Gewinnung der Antikörper und der Zellinien, sowie Verwendung der Antikörper

Es ist seit langem erwünscht, bestimmte oder ausgewählte Proteine anzusprechen bzw. zu diagnostizieren. Dazu bietet es sich an, diese Proteine gezielt mit monoklonalen Antikörpern zu erkennen. Beispielsweise hemmt der humane pankreatisch-sekretorische Trypsininhibitor (h-PSTI) spezifisch h-Trypsin (Serinprotease des Menschen) und spielt deshalb eine wichtige Rolle bei allen Prozessen, bei denen Trypsin inaktive Enzyme durch Spaltung aktiviert. Bei Tumorpatienten, beispielsweise Ovarien-Tumoren (Stenman), ist die Konzentration an freiem, d. h. nicht-trypsin-gebundenem h-PSTI im Serum und im Urin höher als bei Gesunden. Bis heute kann man jedoch nur die Gesamtmenge an h-PSTI (d. h. freiem und enzymgebundenem h-PSTI) im Serum und im Urin mit Hilfe von polyklonalen Antikörpern im Radioimmunassay (RIA) bestimmen; vgl. Clinica Chimica Acta, 103 (1980) 135-143.

Mit Hilfe monoklonaler Antikörper, die beispielsweise gegen das aktive Zentrum von h-PSTI gerichtet sind, ließe sich nun die Konzentration an freiem h-PSTI bestimmen, so daß Tumore diagno stiziert werden könnten. Eine Hybridoma-Zellinie wäre auch eine unerschöpfliche Quelle monoklonaler Antikörper einheitlicher Qualität, eine Voraussetzung für die Zulassung von diagnostischen Testkits.

Die Erfindung betrifft nun gemäß einer Ausführungsform monoklonale Antikörper, die sich zur Diagnose und insbesondere zur quantitativen Bestimmung eines Proteins mit einer vorgegebenen Partialsequenz, beispielsweise dem aktiven Zentrum, oder eines vorgegebenen Haptens verwenden lassen.

Gemäß einer weiteren Ausführungsform betrifft die Erfindung Hybridoma-Zellinien, die sich nach dem im folgenden beschriebenen erfindungsgemäßen Verfahren (I) erhalten lassen und mit deren Hilfe sich wiederum die genannten erfindungsgemäßen monoklonalen Antikörper gewinnen lassen.

Gemäß einer weiteren Ausführungsform betrifft die Erfindung ein mehrstufiges Verfahren (I) zur Gewinnung der genannten erfindungsgemäßen Hybridoma-Zellinien.

Ia) In einer ersten Stufe isoliert man Maus-Milzzellen, ohne sie in B- und T-Lymphozyten aufzutrennen. Zum Begriff und zur Nomenklatur von B- und T-Zellen sowie von T-Helferzellen vgl. man Goding, Monoclonal Antibodies, Principles and Practice, Academic Press Inc. London Ltd., insbesondere Seiten 5 bis 6, 1983, sowie Alberts et al., Molecular Biology of the Cell, Garland Publishing Inc., insbesondere Seiten 994 bis 995, New York & London 1983. Zur gewinnung der Milzzellen kann man BalbC-Mäuse verwenden. Zum Begriff der BalbC-Mäuse sowie zur Milzzellenisolierung und zur im folgenden noch angesprochenen Fusionierung zur Gewinnung von Hybridoma-Zellen vgl. man beispielsweise die deutsche Patentanmeldung P 32 00 657.8.

Ib) Getrennt von der vorhergehenden Stufe sieht man ein Oligopeptid einer bestimmten Sequenz vor, bei der es sich um eine Partialsequenz (beispielsweise das aktive Zentrum) des Proteins handelt, das man später mit erfindungsgemäßen monoklonalen Antikörpern ansprechen will, oder man sieht ein Hapten vor. Diese Oligopeptid oder dieses Hapten kann beispielsweise durch chemische Synthese hergestellt werden. Will man beispielsweise h-PSTI oder h-PSTI-Varianten ansprechen, bei denen das aktive Zentrum des h-PSTI variiert ist, so genügt es, beispielsweise Heptapeptide herzustellen, beispielsweise

```
            17  18  19  20  21  22  23
   NH₂-Thr-Lys-Ile-Tyr-Asn-Pro-Val-COOH  für  PSTI-0       und


            17  18  19  20  21  22  23
   NH₂-Thr-Tyr-Glu-Tyr-Arg-Pro-Val-COOH  für  PSTI-3       und


            17  18  19  20  21  22  23
   NH₂-Thr-Leu-Glu-Tyr-Arg-Pro-Val-COOH  für  PSTI-4.
```

Zur Sequenz von h-PSTI = PSTI-0 vgl. Methods Enzymol., 45 (1976) 813-825 und zur Sequenz bekannter h-PSTI-Varianten vgl. Collins et al., Protein design with human pancreatic secretory trypsin inhibitor, workshop on protease inhibitors, Llubljana 03. 07. 1987 (Anlage).

Ic) In einer weiteren fakultativen Stufe wird das vorgesehene Oligopeptid oder Hapten an einem Träger immobilisiert; zur Immobilisierung von Peptiden an Trägern vgl. man beispielsweise Erlanger, Methods Enzymol., 70 (1980) 85-104. Bei dem Träger kann es sich um ein Trägermolekül handeln, das aus sich wiederholenden gleichen Bausteinen aufgebaut ist, beispielsweise Dextran, Ficoll oder Lipopolysaccharid, mit dem das Oligopeptid mit Hilfe von 1,4-Butandiol-diglycidylether verknüpft werden kann.

Die Tatsache, daß die Immunantwort bei Haptenen (für die Oligopeptide eigentlich nur eine Fallgruppe darstellen), die an Trägermoleküle mit sich wiederholenden Einheiten gebunden sind, in Mäusen unabhängig von T-Zellen und nur eine B-Zellenantwort ist, ist bekannt; vgl. beispielsweise Contemp. Top. Mol. Immunol., 3 (1974) 57-84; J. Exp. Med., 139 (1974) 74-92; J. Immunol., 114 (1975) 704-709; J. Immunol., 114 (1975) 364. Diese Stufe ist also nur fakultativ, so daß das Oligopeptid oder hapten auch in freier Form verwendet werden kann.

Id) In einer weiteren Stufe werden die isolierten Maus-Milzzellen mit dem gegebenenfalls immobilisierten Oligopeptid oder hapten in Gegenwart von

-- Ewing-Sarkoma-Wachstumsfaktor (ESG; für B-Zellen-Proliferation) und

-- monoklonalen Anti-L3/T4-Antikörpern in vitro immunisiert.

Diese Antikörper sind gegen das L3/T4-Antigen auf den T-Helferzellen gerichtet. Demgemäß blockieren die genannten monoklonalen Anti-L3/T4-Antizellen die T-Helferzellen im Zellgemisch der Maus-Milzzellen, so daß es nur zu einer Stimulierung der B-Zellen kommt. Dadurch wird eine unspezifische Stimulierung von T-Helferzellen vermieden und man bekommt in vorteilhafter weise noch vor der folgenden Fusionierungsstufe (Ie) eine sehr effiziente Immunantwort.

Bisher waren nur von T-Zellen abhängige Reaktionen bekannt, so daß die erfindungsgemäße T-Zellenblockierung sowie die mit den B-Zellen erzielbare hohe Anzahl positiver Klone überraschend sind.

Schließlich ist Proc. Natl. Acad. Sci. USA, 81 (1984) 7897-7901 zu entnehmen, daß Immunantworten bisher nur sehr schwer serumfrei erhalten werden konnten.

ESG kann man von Costar Biologicals (Niederlande) beziehen. L3/T4 bezeichnet ein Glycoprotein auf T-Helferzellen(Milzzellen) der Maus. Zur Gewinnung von monoklonalen Anti-L3/T4-Antikörpern vgl. man Nature, 312 (1984) 548 bis 551, Nature 320 (1986) 449 ff. und Cobbold, Thesis, University of Cambridge 1983.

Ie) In einer weiteren Stufe fusioniert man die immunisierten Maus-Milzzellen in an sich bekannter Weise mit Myeloma-Zellen zu Hybridoma-Zellen.

If) Schließlich kloniert man in an sich bekannter Weise die gewonnenen Hybridoma-Zellen.

Gemäß einer weiteren Ausführungsform der Erfindung verwendet man die gewonnenen Hybridoma-Zellen zur Antikörpergewinnung. Zur Herstellung monoklonaler Antikörper vergleiche man dazu allgemein Peters et al., Monoklonale Antikörper, Springer-Verlag, sowie Goding, Monoclonal Antibodies, Principles and Practice, Academic Press Inc. London Ltd., 1983.

Die erfindungsgemäßen monoklonalen Antikörper lassen sich zur Diagnose und insbesondere quantitativen Bestimmung von Proteinen, deren Partialsequenz man bei der Gewinnung der erfindungsgemäßen Hybridoma-Zellinien mit Hilfe des Oligopeptids vorgegeben hat, oder von Haptenen verwenden. Ein Beispiel für eine Verwendung der erfindungsgemäßen monoklonalen Antikörper ist die Tumordiagnose beim Menschen.

Ferner betrifft die Erfindung gemäß einer weiteren Ausführungsform monoklonale Antikörper, die sich zur Diagnose und insbesondere zur quantitativen Bestimmung eines bestimmten Proteins ggf. mit einer vorgegebenen Partialsequenz, beispielsweise dem aktiven Zentrum, verwenden lassen.

Gemäß einer weitern Ausführungsform betrifft die Erfindung Hybridoma-Zellinien, die sich nach dem im folgenden beschriebenen erfindungsgemäßen Verfahren (II) erhalten lassen und mit deren Hilfe sich wiederum die genannten erfindungsgemäßen monoklonalen Antikörper gewinnen lassen.

Gemäß einer weiteren Ausführungsform betrifft die Erfindung ein mehrstufiges Verfahren (II) zur Gewinnung der genannten erfindungsgemäßen Hybridoma-Zellinien.

IIa) In einer ersten Stufe isoliert man Maus-Milzzellen, ohne sie in B- und T-Lymphozyten aufzutrennen. Zum Begriff und zur Nomenklatur von B- und T-Zellen sowie von T-Helferzellen vgl. man Goding, Monoclonal Antibodies, Principles and Practice, Academic Press Inc. London Ltd., insbesondere Seiten 5 bis 6, 1983, sowie Alberts et al., Molecular Biology of the Cell, Garland Publishing Inc., insbesondere Seiten 994 bis 995, New York & London 1983. Zur Gewinnung der Milzzellen kann man BalbC-Mäuse verwenden. Zum Begriff der BalbC-Mäuse sowie zur Milzzellenisolierung und zur im folgenden noch angesprochenen Fusionierung zur Gewinnung von Hybridoma-Zellen vgl. man beispielsweise die deutsche Patentanmeldung P 32 00 657.8.

3

IIb) Getrennt von der vorhergehenden Stufe sieht man ein Protein vor, das man später mit erfindungsgemäßen monoklonalen Antikörpern ansprechen will.

IIc) In einer weiteren Stufe werden die isolierten Maus-Milzzellen mit dem Protein in Gegenwart von

-- Ewing-Sarkoma-Wachstumsfaktor (ESG; für B-Zellen-Proliferation) und

-- peritonealen Mausmakrophagen (die das Protein den B- und T-Zellen optimal präsentieren) in vitro immunisiert.

Wiederum ist Proc. Natl. Acad. Sci. USA, 81 (1984) 7897-7901 zu entnehmen, daß bisher Immunantworten nur sehr schwer serumfrei erhalten werden konnten.

ESG kann man von Costar Biologicals (Niederlande) beziehen. Hinsichtlich peritonealer Makrophagen vgl. Goding, Monoclonal Antibodies, Principles and Practice, Academic Press Inc. London Ltd., insbesondere Seite 71, sowie Peters et al., Monoklonale Antikörper, Springer-Verlag.

IId) In einer weiteren Stufe fusioniert man die immunisierten Maus-Milzzellen in an sich bekannter Weise mit Myeloma-Zellen zu Hybridoma-Zellen.

IIe) Schließlich kloniert bzw. subkloniert man in an sich bekannter Weise die gewonnenen Hybridoma-Zellen.

Gemäß einer weiteren Ausführungsform der Erfindung verwendet man die gewonnenen Hybridoma-Zellen zur Antikörpergewinnung. Durch das vorstehend angesprochene Subklonieren kann man statt eines polyklonalen Gemischs monoklonale Antikörper gewinnen. Han man ein Protein mit einer oder mehreren identifizierbaren (beispielsweise bekannten) Partialsequenzen vorgegeben, so kann man im Elisa- oder RIA-Test ermitteln, ob sich der monoklonale Antikörper gegen die Partialsequenz bzw. gegen welche Partialsequenz er sich richtet.

Die erfindungsgemäßen monoklonalen Antikörper lassen sich zur Diagnose und insbesondere quantitativen Bestimmung von Proteinen verwenden.

Nachstehend wird die Erfindung durch Beispiele näher erläutert.

Beispiele 1 bis 3

Es wurden die Kulturbedingungen für eine in-vitro-Immunisierung mit dem Ziel verbessert, spezifische monoklonale Antikörper gegen die Aminosäuresequenz 17 bis 23 der aktiven Zentren von h-PSTI-0, PSTI-3 und PSTI-4 herzustellen. (Für PSTI und Varianten vgl. EGB 3700204.)

2.5 % (Volumenbasis) Ewing-Sarkoma-Wachstumsfaktor (ESG) beschleunigten die Proliferation der B-Zellklone. Die Immunisierung wurde in Gegenwart von monoklonalen Anti-L3/T4-Antikörpern durchgeführt, um eine unspezifische Proliferation von T-Helferzellen zu verhindern.

Für die Immunisierung wurden die synthetischen Heptapeptide (Aminosäuren 17 bis 23) für PSTI-0, PSTI-3 und PSTI-4 mit 1,4 Butandiol-diglycidylether in einer Menge von etwa 40 mol Peptid/mol Dextran gekoppelt.

Im ELISA und RIA konnte gezeigt werden, daß die erhaltenen Hybridomaklone monoklonale Antikörper produzieren, die spezifisch an die Aminosäuren 17-23 des h-PSTI-0 bzw. der Variante PSTI-3 bzw. PSTI-4 binden. Die monoklonalen Antikörper binden nicht an den PSTI/Protease-Komplex.

Die Hybridoma-Zellinien ware gut produzierende Linien mit 100 $\mu$g Antikörper ml.

Beispiel 4

Es wurden die Kulturbedingungen für eine in-vitro-Immunisierung mit dem Ziel verbessert, spezifische monoklonale Antikörper gegen gentechnologisch erzeugtes humanes Parathormon zu gewinnen.

2.5 % (Volumenbasis) Ewing-Sarkoma-Wachstumsfaktor (ESG) beschleunigten die Proliferation der B-Zellklone. Die Immunisierung wurde ohne monoklonale Anti-L3/T4-Antikörper durchgeführt, da bei einer Immunisierung mit Gesamtproteinen die Proliferation der T-Helfer-Zellen nicht durch die Antikörper blockiert werden darf. Bei der Immunisierung mit Gesamtproteinen sind die B-Zellen und die T-Helfer-Zellen notwendig. Weiterhin wurde die Immunisierung in Gegenwart von peritonealen Maus-Makrophagen durchgeführt (2,5 % x $10^5$ Makrophagen), um das Protein den B- und T-Zellen optimal zu präsentieren.

Für die Immunisierung wurden 10 $\mu$g rekombinantes Parathormon und 5 x $10^7$ Milzzellen eingesetzt; zum h-PTH vgl. P 37 25 320.4.

Nach der Fusion konnte in ELISA gezeigt werden, daß 150 Löcher (von insgesamt 350) der Mikrotiterplatten Klone enthielten, die monoklonale Antikörper gegen das Parathormon produzieren.

Immunisierungsschema:

$5 \times 10^7$ Milzzellen (1/2 Milz von 8 Wochen alten BALB-C-Mäusen)

Immunisierung

Haptene (immobilisiert oder in freier Form)    Proteine

Serumfreies Medium + ESG (Medium A)*    Medium A

+ anti-L3/T4-Antikörper    + peritoneale

5 Tage, 37 °C    Maus-Makrophagen

Medium A ohne ESG, aber 10 % fötales Kälberserum

und 20 µg/ml Lipopolysaccharid

4 Tage, 37 °C

Radioimmunoassay (nach 9 Tagen)

Fusion

*Medium A:

90 % (Volumenbasis) Dulbeccos Modifikation des Eagle-Mediums,

10 % (Volumenbasis) Isocoves Modifikation des Dulbecco-Mediums,

0,3 % $NaHCO_3$, 9 mM Hepes, 2 % (Volumenbasis) Hypoxanthin-ThymidinKonzentrat (50 % HT), 2,1 mM Glutamin, 68,7 µM Streptomycin, 173 µM Ampicillin, 29 µM ß-Mercaptoethanol, 2,5 %

(Volumenbasis) Ewing-Sarcoma-Wachstumsfaktor-Medium (ESG).

**Ansprüche**

1. Monoklonale Antikörper, gewinnbar nach dem Verfahren gemäß Anspruch 6.

2. Hybridoma-Zellinien, gewinnbar nach den Verfahren gemäß einem der Ansprüche 3 bis 5.

3. Verfahren zur Gewinnung der Hybridoma-Zellinien gemäß Anspruch 2, bei dem man

a) Maus-Milzzellen, beispielsweise Milzzellen von BalbC-Mäusen isoliert, ohne sie in B- und T-Lymphozyten aufzutrennen,

b) ein Oligopeptid mit einer bestimmten Sequenz, bei der es sich um eine Partialsequenz eines Proteins handelt, oder ein hapten (jeweils beispielsweise durch chemische Synthese) vorsieht,

c) das Oligopeptid oder Hapten gegebenenfalls an einem Träger immobilisiert,

d) die Maus-Milzzellen mit dem gegebenenfalls immobilisierten Oligopeptid oder Hapten in Gegenwart

-- des Ewing-Sarkoma-Wachstumsfaktors (ESG) und

-- von monoklonalen Anti-L3/T4-Antikörpern in vitro immunisiert,

e) die immunisierten Maus-Milzzellen mit Myelomazellen fusioniert und

f) die gewonnenen Hybridomazellen kloniert.

4. Verfahren nach Anspruch 3, dadurch **gekennzeichnet,** daß man bei der Stufe b) ein Oligopeptid mit dem aktiven Zentrum des pankreatisch-sekretorischen h-Trypsininhibitors oder einer h-Trypsininhibitor-Variante vorsieht, insbesondere einer Variante, bei der das Aktivitätszentrum des h-Trypsininhibitors modifiziert ist.

5. Verfahren nach Anspruch 3 oder 4, dadurch **gekennzeichnet,** daß man bei der Stufe c) an Dextran, Ficoll oder Lipopolysaccharid immobilisiert, insbesondere nach Derivatisierung des Dextrans mit Hilfe von 1.4-Butandiol-diglycidylether.

6. Verfahren zur Gewinnung der monoklonalen Antikörper gemäß Anspruch 1, dadurch **gekennzeichnet,** daß man die gemäß einem der Ansprüche 3 bis 5 gewonnenen Hybridomazellen für die Antikörper-Gewinnung verwendet.

7. Verwendung der monoklonalen Antikörper gemäß Anspruch 1 zur Diagnose, insbesondere zur quantitativen Bestimmung, und zwar eines Proteins mit der partialsequenz gemäß Anspruch 3 Stufe b), beispielsweise im Urin oder Serum, oder eines Haptens.

8. Verwendung nach Anspruch 7 zur Tumordiagnose beim Menschen.

9. Monoklonale Antikörper, gewinnbar nach dem Verfahren gemäß Anspruch 12.

10. Hybridoma-Zellinien, gewinnbar nach dem Verfahren gemäß Anspruch 11.

11. Verfahren zur Gewinnung der Hybridoma-Zellinien gemäß Anspruch 10, bei dem man

a) Maus-Milzzellen, beispielsweise Milzzellen von BalbC-Mäusen isoliert, ohne sie in B- und T-Lymphozyten aufzutrennen,

b) ein Protein vorsieht,

c) die Maus-Milzzellen mit dem Protein in Gegenwart

-- des Ewing-Sarkoma-Wachstumsfaktors (ESG) und

-- von peritonealen Makrophagen immunisiert,

d) die immunisierten Maus-Milzzellen mit Myelomazellen fusioniert und

e) die gewonnenen Hybridomazellen kloniert oder subkloniert.

12. Verfahren zur Gewinnung der monoklonalen Antikörper gemäß Anspruch 9, dadurch **gekennzeichnet,** daß man die gemäß Anspruch 11 gewonnenen Hybridomazellen für die Antikörper-Gewinnung verwendet.

13. Verwendung der monoklonalen Antikörper gemäß Anspruch 9 zur Diagnose, insbesondere zur quantitativen Bestimmung eines Proteins, beispielsweise im Urin oder Serum.

14. Verwendung nach Anspruch 13 zur Tumordiagnose beim Menschen.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | BIOLOGICAL ABSTRACTS/RRM, Nr. 34018869; T.W. BOELDICKE et al.: "A novel strategy to raise monoclonal antibodies against a target site of a protein", & BIOL. CHEM. HOPPE-SEYLER 1987, Band 368, Nr. 9, Seiten 1023-1024 * Der Titel und Term * --- | 1-12 | C 07 K 15/06 C 12 N 5/00 C 12 N 15/00 C 12 P 21/00 G 01 N 33/68 // (C 12 P 21/00 C 12 R 1:91 ) |
| X,P | CHEMICAL ABSTRACTS, Band 109, Nr. 17, 24. Oktober 1988, Seite 560, Nr. 147601z, Columbus, Ohio, US; T. BOELDICKE et al.: "Production of specific monoclonal antibodies against the active sites of human pancreatic secretory trypsin inhibitor variants by in vitro immunization with synthetic peptides", & EUR. J. BIOCHEM. 1988, 175(2), 259-64 * Zusammenfassung * --- | 1-12 | |
| A | US-A-4 444 887 (M.K. HOFFMANN) * Spalte 2, Zeilen 23-46; Ansprüche 1,2 * --- | 3,11 | RECHERCHIERTE SACHGEBIETE (Int. Cl.4)  C 12 P A 61 K C 12 N |
| A | NATURE, Band 301, 13. Januar 1983, Seiten 160-163, Macmillan Journals Ltd; R.F.L.J.S. KONTIAINEN et al.: "A monoclonal antibody against antigen-specific helper factor augments T-cell help" * Insgesamt * ----- | 3,11 | |

Der vorliegende Recherchenbericht wurde für alle Patentansprüche erstellt

| Recherchenort | Abschlußdatum der Recherche | Prüfer |
|---|---|---|
| DEN HAAG | 23-03-1989 | CHARLES D.J.P.I.G. |